# EUROPEAN PATENT APPLICATION

(11) **EP 4 741 301 A1**
(43) Date of publication of application: **13.05.2026**
(21) Application number: 24836099.2
(22) Date of filing: 04.07.2024
(51) Int. Cl.: B65B 55/04, A61L 2/18, A61L 12/06, B65B 55/10

(54) **CONTENT FILLING SYSTEM AND CONTENT FILLING METHOD**

(30) Priority: 04.07.2023 JP 2023110295
(71) Applicant: Dai Nippon Printing Co., Ltd., Tokyo 162-8001 (JP)
(72) Inventor: HAYAKAWA, Atsushi, Tokyo 162-8001 (JP)
(74) Representative: Müller-Boré & Partner Patentanwälte PartG mbB
(86) International application number: PCT/JP2024/024210
(87) International publication number: WO 2025/009587

(57) **Abstract**

[Object] To provide a content filling system having a compact overall configuration.

[Solution] Sterilant is blown from a first sterilization part 193A onto a container 10 molded by a blow molding machine 112, and a label 40 is affixed to the outer periphery of the container 10 in a labeling unit 210. Sterilant is blown from a second sterilization part 193 to the container 10 to which the label 40 has been affixed.

## Description

### Technical Field

The present disclosure relates to a content filling system and a content filling method.

### Background Art

In recent years, containers made of plastic have been widely used as containers for liquid content, such as food or beverage, and such plastic containers are filled with liquid content.

Such plastic containers to be filled with liquid content are manufactured by inserting a preform into a mold and blow-molding the preform. The containers obtained by blow molding are filled with liquid content in an aseptic state.

In a conventionally developed sterilization technique, sterilant is blown onto a preform and a blow-molded container.

After filling, a label indicating the details of the liquid content is affixed to the outer periphery of the container. However, typically, because the label is affixed to the outer periphery of the container after the container is filled with the liquid content in an aseptic state, the overall structure of the aseptic content filling system is large.

### Citation List

### Patent Literature

PTL 1: Japanese Unexamined Patent Application Publication No. 2015-116814
PTL 2: Japanese Unexamined Patent Application Publication No. 2013-133130

### Summary of Invention

### Technical Problem

The present disclosure has been made in view of these circumstances, and provides a content filling system having a compact overall configuration, and a content filling method.

### Solution to Problem

The present disclosure provides a content filling system including: a first sterilization part configured to sterilize at least an outer surface of a container or at least an inner surface of a label; a labeling unit configured to affix the label to the outer surface of the container sterilized by the first sterilization part; a second sterilization part configured to sterilize the container to which the label has been affixed in the labeling unit; a content filling unit configured to fill the container sterilized by the second sterilization part with content; and a sealing unit configured to seal the container.

In the content filling system according to the present disclosure, an air rinse unit configured to blow hot air onto the container is provided between the second sterilization part and the content filling unit.

In the content filling system according to the present disclosure, the second sterilization part blows sterilant onto an inner surface and the outer surface of the container.

In the content filling system according to the present disclosure, the first sterilization part, the labeling unit, and the second sterilization part are enclosed by a first sterilization part chamber, a labeling unit chamber, and a second sterilization part chamber, respectively, so as to be partitioned from each other, and the labeling unit chamber for the labeling unit is at a positive pressure relative to the first sterilization part chamber for the first sterilization part and/or the second sterilization part chamber for the second sterilization part.

In the content filling system according to the present disclosure, the labeling unit includes: a label feeder configured to feed a band-shaped label; a rotary cutter configured to cut the band-shaped label fed from the label feeder to produce the label; a label transport drum that holds by suction the label produced by the rotary cutter; and a star wheel that conveys the container, receives the label held by the label transport drum, and affixes the label to the container. The star wheel, the label transport drum, and the rotary cutter are housed in the labeling unit chamber.

In the content filling system according to the present disclosure, an adhesive application device configured to apply an adhesive or glue to the label held on the label transport drum is provided.

In the content filling system according to the present disclosure, the label feeder is disposed outside the labeling unit chamber, and the band-shaped label is fed from the label feeder into the labeling unit chamber through an opening in the labeling unit chamber.

The present disclosure provides a content filling method including: a first sterilization step of sterilizing, with a first sterilization part, at least an outer surface of a container; a labeling step of affixing, in a labeling unit, a label to the outer surface of the container sterilized by the first sterilization part; a second sterilization step of sterilizing, with a second sterilization part, the container to which the label has been affixed in the labeling unit; a content filling step of filling, in a content filling unit, the container sterilized by the second sterilization part with content; and a sealing step of sealing the container in a sealing unit.

In the content filling method according to the present disclosure, hot air is blown onto the container by an air rinse unit provided between the second sterilization part and the content filling unit.

In the content filling method according to the present disclosure, the second sterilization part blows sterilant onto an inner surface and the outer surface of the container.

In the content filling method according to the present disclosure, the first sterilization part, the labeling unit, and the second sterilization part are enclosed by a first sterilization part chamber, a labeling unit chamber, and a second sterilization part chamber, respectively, so as to be partitioned from each other, and the labeling unit chamber for the labeling unit is at a positive pressure relative to the first sterilization part chamber for the first sterilization part and/or the second sterilization part chamber for the second sterilization part.

In the content filling method according to the present disclosure, the labeling step includes: a step of feeding a band-shaped label from a label feeder; a step of cutting, with a rotary cutter, the band-shaped label fed from the label feeder to produce the label; a step of holding by suction and transporting, with a label transport drum, the label produced by the rotary cutter; and a step of conveying the container, receiving the label held by the label transport drum, and affixing the label to the container with a star wheel. The star wheel, the label transport drum, and the rotary cutter are housed in the labeling unit chamber.

In the content filling method according to the present disclosure, an adhesive is applied to the label held on the label transport drum by an adhesive application device.

In the content filling method according to the present disclosure, the label feeder is disposed outside the labeling unit chamber, and the band-shaped label is fed from the label feeder into the labeling unit chamber through an opening in the labeling unit chamber.

### Advantageous Effects of Invention

According to the present invention, it is possible to provide a content filling system having a compact configuration, which can sterilize containers and attach labels to the outer peripheries of the containers.

### Brief Description of Drawings

[Fig. 1] Fig. 1 is a partial vertical sectional view of a container used in an embodiment.
[Fig. 2] Fig. 2 is a horizontal sectional view (sectional view taken along line II-II in Fig. 1) showing a container used in the embodiment.
[Fig. 3] Fig. 3 is a vertical sectional view of a preform used in the embodiment.
[Fig. 4] Fig. 4 is a sectional view of a label.
[Fig. 5] Figs. 5(A) to (C) are schematic views showing a preform sterilization step.
[Fig. 6] Figs. 6(D) to (F) are schematic views showing a preform sterilization step and a container molding step.
[Fig. 7] Figs. 7(G1) to (H) are schematic views showing a container sterilization step and a content filling step.
[Fig. 8] Figs. 8(I) to (J2) are schematic views showing a container sterilization step.
[Fig. 9] Figs. 9(K) to (M) are schematic views showing a container sterilization step, a content filling step, and a cap-sealing step.
[Fig. 10A] Fig. 10A is a schematic view of a content filling system.
[Fig. 10B] Fig. 10B is a schematic view of a labeling unit.
[Fig. 10C] Fig. 10C shows a modification of the labeling unit.
[Fig. 11] Fig. 11 shows a spindle in a heating furnace.

### Description of Embodiments

### Embodiments

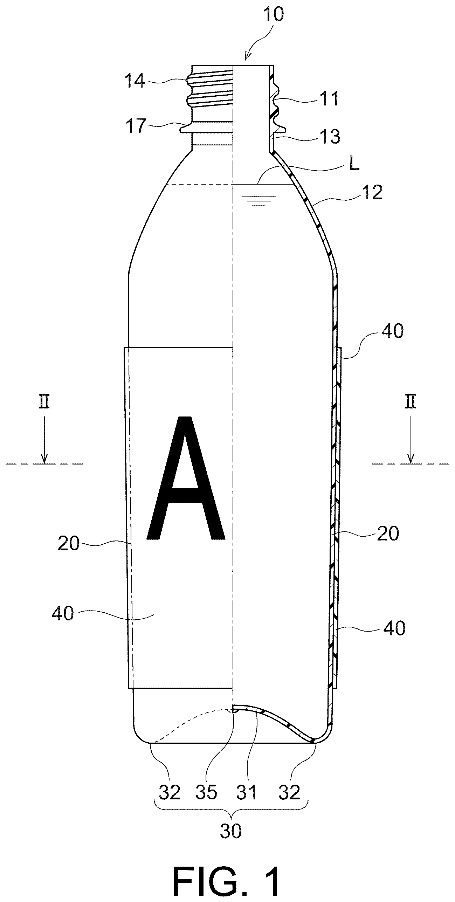

Hereinbelow, an embodiment of the present invention will be described with reference to the drawings. Figs. 1 to 11 show an embodiment of the present invention.

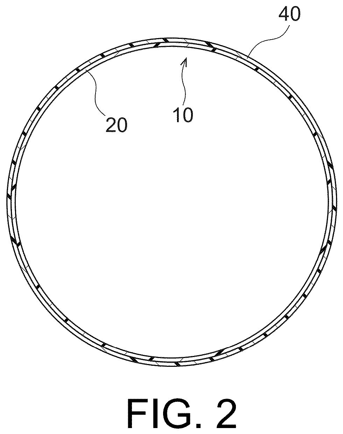

First, an overview of a container used in an aseptic content filling system according to this embodiment will be described with reference to Figs. 1 and 2. In the present specification, "top" and "bottom" respectively refer to the upper side and the lower side in a state in which a container 10 is erected (Fig. 1).

As described below, the container (plastic bottle) 10 shown in Figs. 1 and 2 is obtained by performing biaxial stretch blow molding on a preform 10a using a blow molding mold 104 to expand the preform 10a. The container 10 is filled with liquid content (also referred to as content) L, and a liquid-filled container 10A is obtained.

This container 10 is made of a plastic material.

The container 10 includes a mouth portion 11, a neck portion 13 provided below the mouth portion 11, a shoulder portion 12 provided below the neck portion 13, a body portion 20 provided below the shoulder portion 12, and a bottom portion 30 provided below the body portion 20.

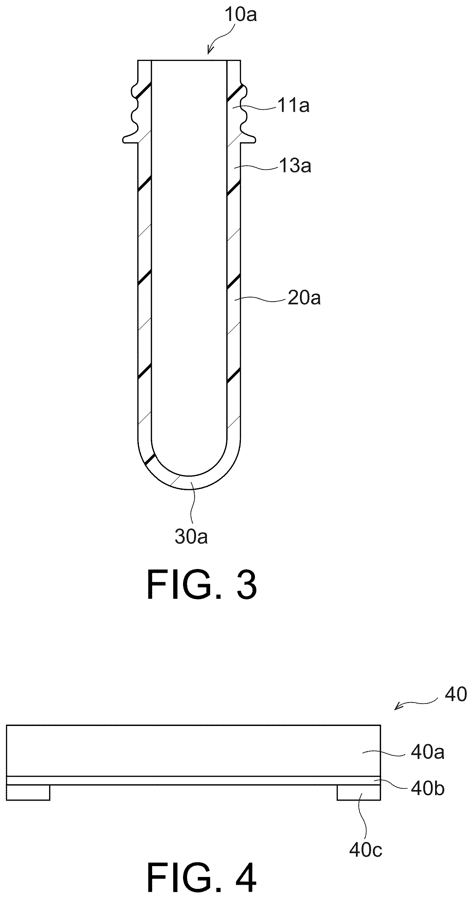

A label 40 indicating the product name, graphics, or characteristics such as ingredients or effects of the liquid content is affixed to the body portion 20 of the container 10. As shown in Fig. 4, the label 40 includes a label body 40a and a print layer 40b located on the container 10 side and including graphics and text. Of these, the label body 40a has a multilayer structure or a single-layer structure including, for example, a plastic layer or a paper layer. An adhesive 40c is applied to the print layer 40b.

Next, the container 10 will be described in detail. As described above, the container 10 has the mouth portion 11, the neck portion 13, the shoulder portion 12, the body portion 20, and the bottom portion 30.

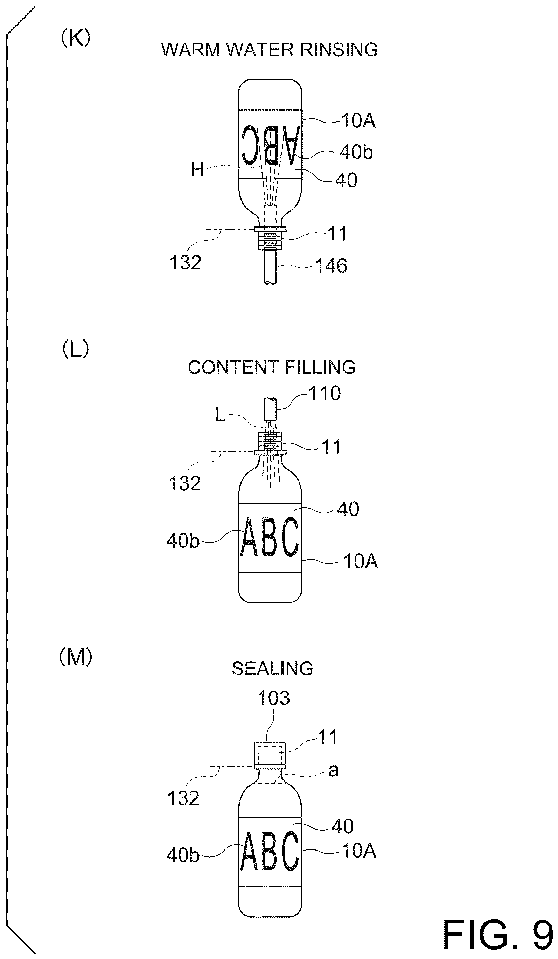

The mouth portion 11 has a threaded portion 14 to be engaged with a cap 103 (see Fig. 9(M)) and a support portion 17 provided below the threaded portion 14. The shape of the mouth portion 11 may be a known shape.

The neck portion 13 is located between the support portion 17 and the shoulder portion 12, and has a substantially cylindrical shape with a substantially uniform diameter. The shoulder portion 12 is located between the neck portion 13 and the body portion 20, and has such a shape that the diameter thereof gradually increases from the neck portion 13 side toward the body portion 20 side.

Furthermore, the body portion 20 has a cylindrical shape with a substantially uniform diameter as a whole. However, the present invention is not limited thereto, and the body portion 20 may have a polygonal tubular shape, such as a quadrangular tubular shape or an octagonal tubular shape. Alternatively, the body portion 20 may have a tubular shape with a non-uniform horizontal cross section from the upper side to the lower side. In this embodiment, the body portion 20 has a substantially flat surface without irregularities, but the present invention is not limited thereto. For example, the body portion 20 may have irregularities such as panels or grooves.

The bottom portion 30 has a recess 31 located at the center and a ground contact portion 32 provided around the recess 31. The shape of the bottom portion 30 is not particularly limited, and the bottom portion may have a known shape (for example, a petaloid bottom shape, a round bottom shape, or the like). A gate portion 35, which serves as a path through which resin is injected into the mold when the preform (including a parison) 10a is produced by injection molding, is formed at the center of the bottom portion 30. The gate portion 35 has a thickness of about 0.5 mm to 5.0 mm.

The thickness of the container 10 at the body portion 20 may be reduced to, for example, about 50 µm to 250 µm, although it is not limited thereto. Furthermore, the weight of the container 10 may be set to 10 g to 30 g for a 500 mL bottle, although it is not limited thereto. By reducing the wall thickness of the container 10 in this way, the weight reduction of the container 10 is achieved.

This container 10 can be manufactured by biaxially stretch-blow molding the preform 10a (described below), which has been manufactured by injection-molding a synthetic resin material. It is preferable to use a thermoplastic resin, particularly polyethylene (PE), polypropylene (PP), polyethylene terephthalate (PET), polyethylene naphthalate (PEN), or polycarbonate (PC), as a material for the preform 10a (i.e., for the container 10). These various resins may be blended for use. Furthermore, to improve the barrier properties of the container, a vapor-deposited film, such as a diamond-like carbon film or a silicon oxide thin film, may be formed on the inner surface of the container 10.

The container 10 may also be formed as a multilayer molded bottle having two or more layers. That is, a preform 10a having three or more layers, an intermediate layer of which is made of a resin (intermediate layer) having gas barrier properties and light-shielding properties, such as nylon MXD6 (hereinafter also referred to as MXD6), MXD6 + fatty acid salt, polyglycolic acid (PGA), ethylene vinyl alcohol copolymer (EVOH), or polyethylene naphthalate (PEN), may be produced by extrusion molding or injection molding. Then, the preform 10a is blow-molded into a multilayer bottle having gas barrier properties and light-shielding properties. The intermediate layer may be made of a resin obtained by blending the various resins described above.

Alternatively, a foamed preform having a foam cell diameter of 0.5 µm to 100 µm may be produced by mixing an inert gas (nitrogen gas or argon gas) with a melt of a thermoplastic resin, and the foamed preform may be blow-molded to produce the container 10. Because this container 10 contains the foam cells therein, the light-shielding property of the overall container 10 is enhanced.

This container 10 may be a bottle with a full capacity of 150 mL to 1500 mL, for example.

The label 40 is affixed to the body portion 20 of the container 10 as described above, and has the label body 40a made of a film and indicating the characteristics and the like of the liquid content to fill the container 10, the print layer 40b provided on one surface of the label body 40a, and the adhesive 40c, such as hot melt, applied to the print layer 40b. The label 40 is then affixed to the body portion 20 of the container 10 via the adhesive 40c. An adhesive application device 212A described below applies the adhesive 40c to the label 40. However, the label 40 may be a thermal label, a shrink label, a stretch label, a stretch shrink label, a tag label, or the like disposed on the outer periphery of the body portion 20 of the container 10. The thickness of the label 40 is 5 µm to 150 µm, and preferably 10 µm to 80 µm. Examples of resins forming the label 40 include polyolefin resins (e.g., polyethylene resins and polypropylene resins), polyester resins, polystyrene resins, polyvinyl chloride resins, and polyamide resins. Any one of the above resins may be used alone, or two or more kinds thereof may be used in combination, and a composite label made of a resin other than the above resins and another material may also be used.

The label 40 extends from the lower portion to the center of the body portion 20 of the container 10 and terminates short of the lower end of the shoulder portion 12 (see Fig. 1).

Next, referring to Fig. 3, the configuration of the preform used in this embodiment will be described.

As shown in Fig. 3, the preform 10a is made of a plastic material.

The preform 10a includes a mouth portion 11a, a body portion 20a connected to the mouth portion 11a, and a bottom portion 30a connected to the body portion 20a. The mouth portion 11a corresponds to the mouth portion 11 of the container 10 described above, and has substantially the same shape as the mouth portion 11. The body portion 20a corresponds to the neck portion 13, the shoulder portion 12, and the body portion 20 of the container 10 described above, and has a substantially cylindrical shape. The bottom portion 30a corresponds to the bottom portion 30 of the container 10 described above, and has a substantially hemispherical shape.

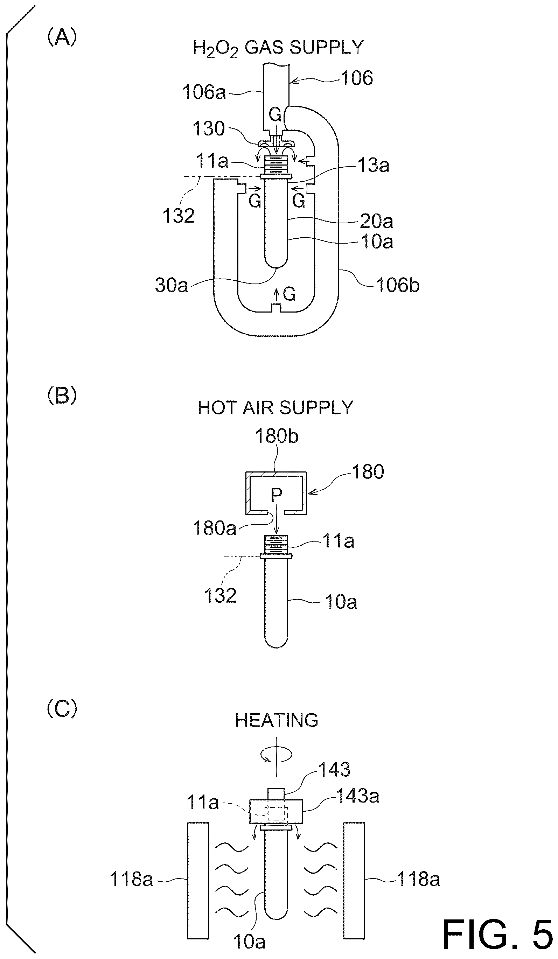

Next, an aseptic content filling system and an aseptic content filling method according to the present disclosure will be described with reference to Figs. 5A to 11.

As shown in Figs. 5(A) to 9(M), the preform 10a and the container 10 are subjected to a sterilization treatment, and the container 10 is filled with liquid content L to obtain a liquid-filled container 10A.

First, the preform 10a shown in Fig. 5(A) is continuously conveyed at a desired speed, and sterilant gas G, mist, or a mixture thereof is blown onto the inner surface and outer surface of the traveling preform 10a and is adsorbed.

In this embodiment, hydrogen peroxide is used as the sterilant, but another sterilant may also be used.

In this case, as shown in Fig. 5(A), gas G of hydrogen peroxide, serving as sterilant, is blown from a sterilant supply nozzle 106 to the preform 10a.

The hydrogen peroxide gas G is divided into two flows within the sterilant supply nozzle 106, one of which is jetted from a nozzle main pipe 106a into the preform 10a, and the other of which is jetted from a nozzle branch pipe 106b toward the outer surface of the preform 10a. After exiting the sterilant supply nozzle 106, the hydrogen peroxide gas G, still in a gaseous state, in a mist form, or as a mixture thereof, flows into the preform 10a or comes into contact with the outer surface of the preform 10a.

The flow of the gas G jetted into the preform 10a is covered with an umbrella-shaped member 130. The gas G or mist flowing into the preform 10a flows out from the mouth portion 2a of the preform 10a. The outflowing gas G impinges on the umbrella-shaped member 130, is guided along the inner surface of the umbrella-shaped member 130, is redirected toward the outer surface of the preform 10a, and comes into contact with the outer surface of the preform 10a.

In this case, as a result of the gas G, mist, or a mixture thereof containing a hydrogen peroxide component, serving as sterilant, coming into contact with and deposited on the inner and outer surfaces of the preform 10a, microorganisms on the surfaces of the preform 10a are killed or damaged. As the sterilant, besides hydrogen peroxide, ozonated water, one containing a peracetic acid component, or one containing a chlorine component may be used. Any sterilant that inactivates microorganisms may be used.

Immediately before the gas G is blown onto the preform 10a as shown in Fig. 5(A), hot air may be blown onto the preform 10a to preheat the preform.

Although an example in which the sterilant gas G is blown onto the inner surface and outer surface of the preform 10a has been described, the sterilant gas G may be blown only onto the inner surface of the preform 10a.

Next, by using an air nozzle 180 having a nozzle body 180b, hot air P is supplied into the preform 10a through an opening 180a of the nozzle body 180b (Fig. 5(B)).

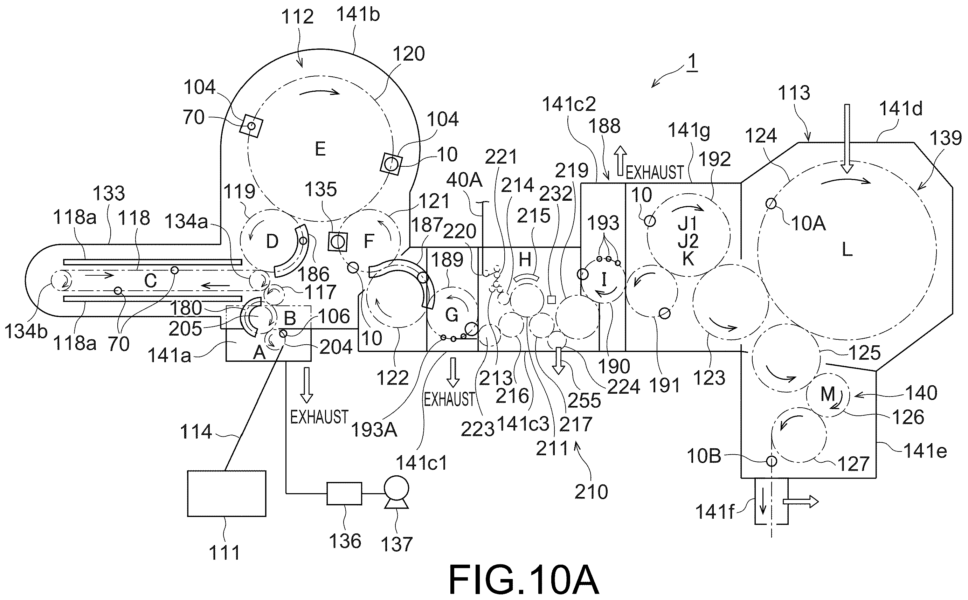

By blowing the hot air P, the hydrogen peroxide deposited on the inner surface of the preform 10a is activated by the heat of the hot air P, whereby the microorganisms in the preform 10a are killed. In addition, blowing of the hot air P quickly removes the hydrogen peroxide deposited on the inner surface of the preform 10a from the surface of the preform 10a. As described below, both the sterilant supply nozzle 106 and the air nozzle 180 are disposed in a chamber 141a (see Fig. 10A).

As shown in Fig. 5(C), the sterilized preform 10a is heated to a temperature suitable for subsequent blow molding by infrared heaters 118a or other heating means. This temperature is about 90 °C to 130 °C. To prevent deformation or the like, the mouth portion 11a of the preform 10a passes above the area directly facing the infrared heaters 118a, so that the heat from the infrared heaters 118a is not transmitted thereto.

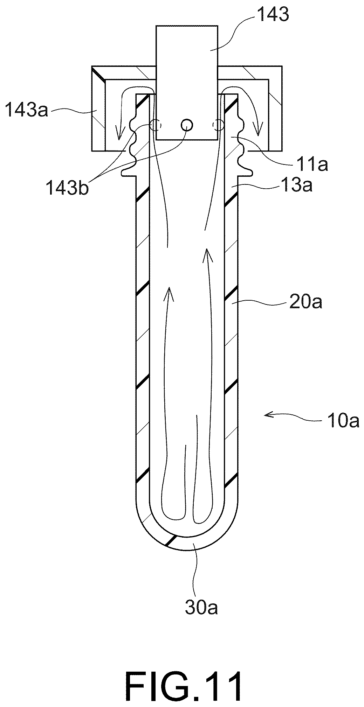

As shown in Fig. 5(C), when the preform 10a is heated, the preform 10a is supported by a spindle 143. The state in which the preform 10a is supported by the spindle 143 will be described in detail with reference to Fig. 11.

As shown in Fig. 11, a plurality of ball-shaped elastic bodies 143b are embedded in the lower portion of the spindle 143. In addition, an umbrella-shaped member 143a is attached to the outside of the spindle 143 as necessary. Fig. 11 shows the spindle 143 in a heating furnace 133.

When the lower portion of the spindle 143 is inserted into the mouth portion 11a, the preform 10a is supported by the spindle 143 by means of elastic deformation of the elastic bodies 143b. When the umbrella-shaped member 143a is provided, the mouth portion 11a of the preform 10a is covered with the umbrella-shaped member 143a at the same time.

As shown in Fig. 11, when the umbrella-shaped member 143a is provided, a gap is formed from a portion between the inner surface of the mouth portion 11a of the preform 10a and the lower portion of the spindle 143 to a portion between the outer surface of the mouth portion 11a of the preform 10a and the umbrella-shaped member 143a. The air in the preform 10a heated by the infrared heaters 118a becomes hot air and flows through the gap, from the inside of the preform 10a to the outside of the preform 10a, while heating the mouth portion 11a of the preform 10a. At the same time, the body portion 20a of the preform 10a is heated by the heat from the infrared heaters 118a, and the heat of the body portion 20a is transferred to the mouth portion 11a. In this way, as heat is transferred from the body portion 20a to the mouth portion 11a, the mouth portion 11a is heated, activating the hydrogen peroxide deposited on the inner and outer surfaces of the mouth portion 11a, thus killing the microorganisms present on the inner and outer surfaces of the mouth portion 11a. In this embodiment, heat transferred from the body portion 20a to the mouth portion 11a more effectively heats the mouth portion 11, compared with the case where the hot air in the preform 10a heats the mouth portion 11a. This effectively raises the temperature of the mouth portion 11a.

The mouth portion 11a of the preform 10a is designed not to be deformed by the heat applied at the stage of the preform 10a, in order to maintain the airtightness of the container 10 when the container 10 is sealed with a cap 103 in a subsequent step.

The hot air flowing through the gap heats the mouth portion 11a only to a temperature of not more than about 70 °C, at which the mouth portion 11a is not deformed. By heating the mouth portion 11a in this way, a trace amount of residual hydrogen peroxide in the preform 10a is activated, appropriately sterilizing the mouth portion 11a.

In order to improve the sterility level of the mouth portion 11a, the mouth portion 11a may be irradiated and sterilized with a UV lamp, a pulsed beam device, or the like in addition to the hot air guided by the umbrella-shaped member 143a. In this case, the UV lamp or the pulsed beam device may be provided upstream of the sterilant supply nozzle 106 or downstream of the infrared heaters 118a, or may be provided both upstream of the sterilant supply nozzle 106 and downstream of the infrared heaters 118a.

During heating, the preform 10a is conveyed, preferably, while axially rotating together with the spindle 143, in a state of being suspended in an upright state by the spindle 143 inserted into the mouth portion 11a thereof. This allows the preform 10a to be uniformly heated to about 90 °C to 130 °C by the infrared heaters 118a, except for the mouth portion 11a.

The preform 10a may be conveyed in an inverted state.

During this, the preform 10a is heated by the infrared heaters 118a, and the hydrogen peroxide remaining on the outer surface of the preform 10a is also activated. Hence, the outer surface of the preform 10a can be appropriately sterilized by the activated hydrogen peroxide.

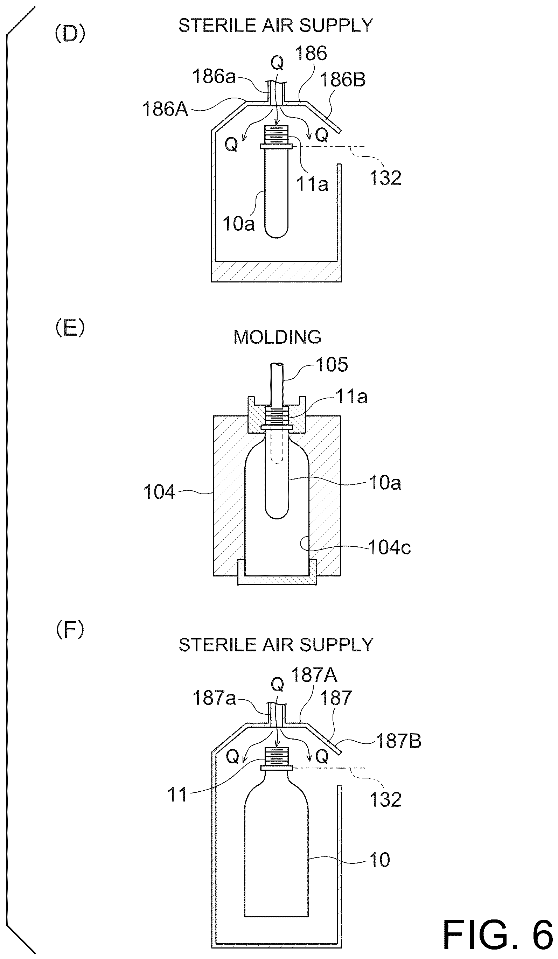

As shown in Fig. 6(D), the heated preform 10a is released from the spindle 143, transferred to a gripper 132, and conveyed to the mold 104, which is the blow molding mold constituting a blow molding machine 112 shown in Fig. 6(E), while being subjected to blowing of sterile air Q from the mouth portion 11a thereof. The preform 10a, onto which the sterile air Q has been blown, is supplied to the mold 104 while maintaining a high level of sterility.

The sterile air Q may be hot air. Blowing hot air prevents a decrease in the temperature of the preform 10a.

As shown in Fig. 6(D), a tunnel-shaped cover 186 is provided so as to surround the traveling path of the preform 10a at a position where the heating of the preform 10a is finished and the preform 10a is directed to the mold 104. The tunnel-shaped cover 186 has a ceiling portion 186A that covers the mouth portion 11a of the preform 10a from above, and the ceiling portion 186A is formed in a roof shape having an inclined surface 186B. The ceiling portion 186A is provided with nozzles 186a, as a row of pipes or as a slit, that discharge the sterile air Q toward the mouth portion 11a of the preform 10a. As described below, both the molds 104 and the cover 186 are disposed in the chamber 141b (see Fig. 10A). With this configuration, the sterile air Q is efficiently supplied to the preform 10a, and the preform 10a in the chamber 141b travels while being maintained in a sterilized state. Alternatively, microbial contamination can be reduced by setting the chamber 141b, which houses the blow molding machine 112, to Class 100,000 or lower, preferably Class 10,000 or lower, without providing the tunnel-shaped cover 186.

The preform 10a onto which the sterile air Q has been blown and which is conveyed while being maintained in a sterilized state is accommodated in the mold 104, as shown in Fig. 6(E).

The mold 104 is brought into a clamped state while continuously traveling at the same speed as the traveling speed of the preform 10a, and is brought into an open state after the preform 10a is blow-molded in the mold 104.

As described above, in the heating step shown in Fig. 5(C), the entire preform 10a except for the mouth portion 11a is uniformly heated to a temperature range suitable for molding. Hence, as shown in Fig. 6(E), when a stretch rod 105 is inserted into the preform 10a after the heated preform 10a is mounted in the mold 104, the preform 10a is stretched in the mold 104 in the longitudinal direction thereof.

Subsequently, for example, sterile air for primary blowing and sterile air for secondary blowing are sequentially blown into the preform 10a through a blow nozzle (not shown), whereby the preform 10a expands to become a container (plastic bottle) 10, serving as a molded article, in the cavity 104c of the mold 104.

Once the container 10 is molded in the mold 104 in this way, the mold 104 is opened while continuously traveling, and the finished container 10 is taken out from the mold 104 to the outside (container molding step).

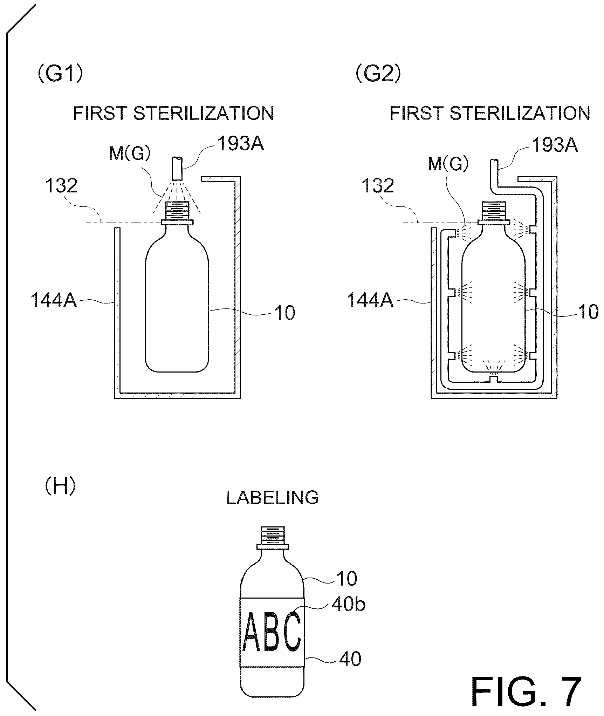

After being taken out from the mold 104 to the outside and until a hydrogen peroxide supply step shown in Fig. 7(G) is reached, the container 10 is conveyed while being subjected to blowing of the sterile air Q from the mouth portion 11 side, as shown in Fig. 6(F). The container 10 is conveyed to a position immediately below sterilant supply nozzles 193A, which supply hydrogen peroxide, while being subjected to blowing of the sterile air Q to avoid microbial contamination as much as possible.

The sterile air Q shown in Fig. 6(F) is preferably hot air. Because blowing of hot air prevents a decrease in the temperature of the container 10, the sterilization effect by the following hydrogen peroxide step is improved.

As shown in Fig. 6(F), a tunnel-shaped cover 187 is provided so as to surround the traveling path of the container 10, at a position where the container 10 moves to the subsequent sterilant supply nozzles 193A (see Fig. 7(G)). The tunnel-shaped cover 187 has a ceiling portion 187A that covers the mouth portion 11 of the container 10 from above, and the ceiling portion 187A is formed in a roof shape having an inclined surface 187B. The ceiling portion 187A is provided with nozzles 187a, as a row of pipes or as a slit, that discharge the sterile air Q toward the mouth portion 11 of the container 10 or toward the traveling path. Thus, the sterile air Q is efficiently supplied to the container 10. As shown in Fig. 10A, an inspection device 135 for inspecting the appearance of the molded container 10 is provided adjacent to the cover 187. The inspection device 135 is disposed within the chamber 141b, and the cover 187 extends from the chamber 141b into a chamber 141c1. As described, the container 10 travels through the chambers 141b and 141c1 (described below) with minimized microbial contamination of the sterilized container 10 (see Fig. 10A). Alternatively, microbial contamination may be reduced by setting the chamber 141b, which houses the blow molding machine 112, to Class 100 or higher and Class 100,000 or lower, preferably Class 10,000 or lower, without providing the tunnel-shaped cover 187.

Next, as shown in Fig. 7(G1), hydrogen peroxide, serving as sterilant, is supplied to the container 10, onto which the sterile air Q has been blown, to sterilize at least the outer surface of the container 10.

In particular, the hydrogen peroxide mist M, gas G, or a mixture thereof is blown from the sterilant supply nozzles 193A (first sterilization part) onto the outer surface, including at least the portion to which the label 40 is affixed, and onto the inner surface of the container 10 being held by the gripper 132 and conveyed (first sterilization step). The sterilant supply nozzles 193A are disposed so as to face the mouth portion 11 of the container 10. The hydrogen peroxide mist M, gas G, or a mixture thereof flows down from the tips of the sterilant supply nozzles 193A and comes into contact with the outer surface and the inner surface of the container 10 (see Fig. 7(G1)). After exiting the sterilant supply nozzles 193A, the hydrogen peroxide gas G, still in a gaseous state, in a mist form, or as a mixture thereof, comes into contact with the outer surface and the inner surface of the container 10. As a result of the gas G, mist, or a mixture thereof containing a hydrogen peroxide component as sterilant coming into contact with and being deposited on the outer surface and the inner surface of the container 10, microorganisms on the outer surfaces of the container 10 are killed or damaged. As the sterilant, besides hydrogen peroxide, one containing a peracetic acid component may be used. Similarly to the sterilant supply nozzle 106 shown in Fig. 5(A), the sterilant supply nozzles 193A may be divided into two so as to spray the sterilant not only onto the outer surface of the container 10 but also onto the inner surface of the container 10.

Furthermore, a tunnel 144A is formed at the position where the container 10 travels, and the hydrogen peroxide mist M, gas G, or a mixture thereof blown from the sterilant supply nozzles 193A flows down along the outer surface of the container 10 and stays in the tunnel 144A, so that the mist, gas, or a mixture thereof effectively deposits on the outer surface of the container 10.

The hydrogen peroxide mist M or gas G can be generated by, for example, a sterilant gas generator.

The sterilant supply nozzles 193A may be disposed at a fixed position on the conveying path of the container 10, or may be moved in synchronization with the container 10.

As shown in Fig. 7(G1), the hydrogen peroxide mist M, gas G, or a mixture thereof discharged from the sterilant supply nozzles 193A contacts the inner and outer surfaces of the container 10. At this time, because the container 10 is kept at a predetermined temperature by the residual heat applied to the container 10 at the stage of the preform 10a and at the stage shown in Fig. 6(F), the container 10 is efficiently sterilized.

When the preform 10a is made of PET, this predetermined temperature is preferably 40 °C to 80 °C, more preferably 50 °C to 75 °C. When the temperature is lower than 40 °C, the sterility decreases. When the temperature is higher than 80 °C, a problem that the container 10 shrinks after molding may occur.

Instead of blowing the hydrogen peroxide mist M, gas G, or a mixture thereof onto both the outer surface and the inner surface of the container 10 as shown in Fig. 7(G1), the hydrogen peroxide mist M, gas G, or a mixture thereof may be blown only onto the outer surface of the container 10 from the sterilant supply nozzles 193A as shown in Fig. 7(G2). In particular, the sterilant supply nozzles 193A may be extended along the body portion 20 and the bottom portion 30 of the container 10 in the tunnel 144A, and the hydrogen peroxide mist M, gas G, or a mixture thereof may be horizontally blown from the sterilant supply nozzles 193A toward the outer surface of the body portion 20. Furthermore, the hydrogen peroxide mist M, gas G, or a mixture thereof may be blown upward from the sterilant supply nozzles 193A toward the outer surface of the bottom portion 30. When the liquid content is mineral water, the sterilant may be replaced with water, and water vapor may be blown onto the container 10. Alternatively, the preform 10a may be conveyed to the heating furnace 133 without hot air P being blown.

A first sterilization step using the sterilant supply nozzles 193A (first sterilization part) may be performed only on the outer surface of the container 10 to which the label 40 is to be affixed, as described above.

Next, as shown in Fig. 7(H), the container 10, onto the inner surface and outer surface of which the hydrogen peroxide mist M, gas G, or a mixture thereof has been blown, is sent to a labeling unit 210, and, in the labeling unit 210, the label 40 is affixed to the body portion 20 of the container 10, in the region from the lower portion through the central portion to the portion below the shoulder portion 12 (labeling step).

In this manner, the label 40 is affixed not to the entire region of the body portion 20 of the container 10, but to a region of about 60%, from the lower portion through the central portion to the portion below the shoulder portion 12.

As described above, the label 40 includes the label body 40a and the print layer 40b provided on the label body 40a and indicating the characteristics of the liquid content to fill the container 10.

The labeling unit 210 includes a star wheel 211 disposed in a chamber 141c3, a label transport drum 212, the adhesive application device 212A, and a label pressing device 215. The detailed structure of the labeling unit 210 will be described below.

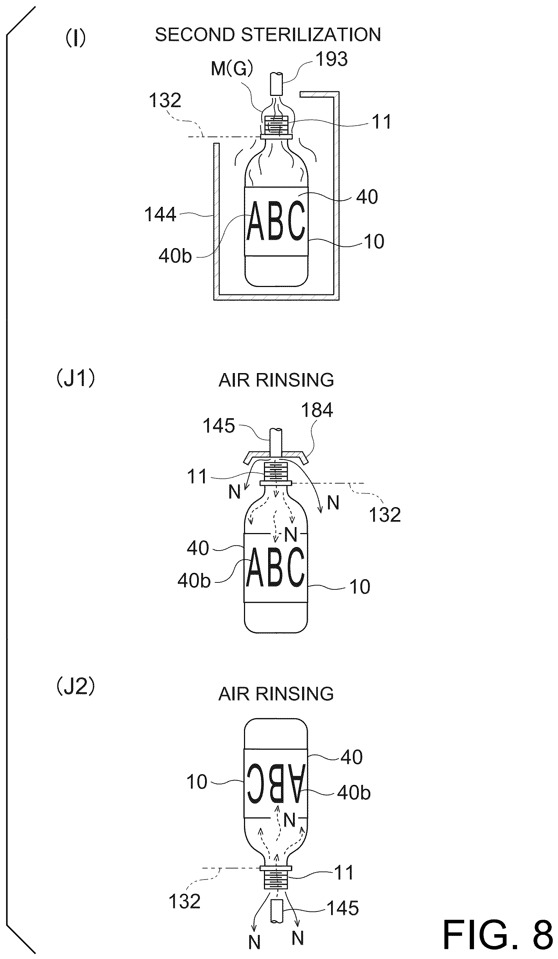

Next, as shown in Fig. 8(I), the container 10 to which the label 40 has been affixed is sterilized by supplying hydrogen peroxide, serving as sterilant, to the container 10.

In particular, the hydrogen peroxide mist M, gas G, or a mixture thereof is blown from the sterilant supply nozzles 193 (second sterilization part) to the container 10 that is being held by the gripper 132 and conveyed (second sterilization step). The sterilant supply nozzles 193 are disposed so as to face the mouth portion 11 of the container 10. The hydrogen peroxide mist M, gas G, or a mixture thereof flows down from the tip of the sterilant supply nozzles 193, enters the container 10 from the mouth portion 11 of the container 10, and comes into contact with the inner surface of the container 10.

Furthermore, a tunnel 144 is formed at the position where the container 10 travels, and the hydrogen peroxide mist M, gas G, or a mixture thereof discharged from the sterilant supply nozzles 193 flows down along the outer surface of the container 10 and the outer surface of the label 40 and stays in the tunnel 144, so that the mist, gas, or a mixture thereof effectively deposits on the outer surface of the container 10 and the outer surface of the label 40.

The hydrogen peroxide mist M or gas G can be generated by, for example, a sterilant gas generator.

The sterilant supply nozzles 193 may be disposed at a fixed position on the conveying path of the container 10, or may be moved in synchronization with the container 10.

As shown in Fig. 8(I), the hydrogen peroxide mist M, gas G, or a mixture thereof discharged from the sterilant supply nozzles 193 contacts the inner and outer surfaces of the container 10 and the outer surface of the label 40.

After the hydrogen peroxide mist M, gas G, or a mixture thereof is blown, the container 10 is air-rinsed as shown in Fig. 8(J1). Air rinsing is performed by blowing sterile air N from a nozzle 145 into the container 10. The flow of the sterile air N removes foreign matter, hydrogen peroxide, and the like from inside the container 10. At this time, the container 10 is in an upright state.

Preferably, an umbrella-shaped member 184 is attached to the nozzle 145. By the guide action of the umbrella-shaped member 184, the sterile air N overflowing from the container 10 flows toward the outer surface of the container 10, air-rinsing the outer surface of the container 10.

Furthermore, supplying heated sterile air N from the nozzle 145 to the container 10 can activate the hydrogen peroxide supplied in the second sterilization step and remaining on the inner surface and outer surface of the container 10 and the outer surface of the label 40. At the same time, the hydrogen peroxide supplied in the first sterilization step and remaining between the container 10 and the label 40 can be activated.

Instead of the air rinsing step in Fig. 8(J1), an air rinsing step as shown in Fig. 8(J2) may be adopted. In the step as shown in Fig. 8(J2), by inverting the container 10 and blowing the sterile air N into the container 10 from the mouth portion 11 facing downward, foreign matter or the like in the container 10 can be caused to drop out of the container 10 through the mouth portion 11. Alternatively, the step in Fig. 8(J2) may be performed following the air rinsing step in Fig. 8(J1), without the sterile air N being blown. The umbrella-shaped member 184 may also be attached to the nozzle 145 shown in Fig. 8(J2). Furthermore, a dilute hydrogen peroxide gas may be added to the hot air used in the air rinsing step to improve the sterilization effect. Specifically, hydrogen peroxide mist or gas is added to hot air at 70 °C to 170 °C. The hydrogen peroxide used at this time may have a gas concentration of 0.5 mg/L to 5 mg/L.

After the air rinsing, as shown in Fig. 9(K), if necessary, sterile water rinsing is performed with sterile room-temperature water or hot water of 15 °C to 85 °C using a nozzle 146 to wash away hydrogen peroxide deposited on the container 10 and remove foreign matter. It is desirable that the flow rate per nozzle 146 be 5 L/min to 15 L/min, and that the rinsing time be 0.2 seconds to 10 seconds.

As described above, because the container 10 is further sterilized with hydrogen peroxide after it has been sterilized at the stage of the preform 10a, the amount of use of hydrogen peroxide for the container 10 may be small, and hence, the warm water rinsing step after the air rinsing can be omitted.

The hydrogen peroxide mist M or gas G used in the first sterilization step shown in Fig. 7(G1) or Fig. 7(G2) and the second sterilization step shown in Fig. 8(I) is as follows.

When the amount of use of hydrogen peroxide is converted to the amount of mist M, it is usually necessary that hydrogen peroxide in an amount of 50 µL to 100 µL per 500 mL bottle, in terms of 35% by weight, be deposited on the container 10 to sterilize the container 10. However, when the preform 10a is sterilized as in this embodiment, in both the case of the first sterilization step as shown in Fig. 7(G1) or Fig. 7(G2) and the case of the second sterilization step as shown in Fig. 8(I), commercial aseptic filling is enabled by allowing hydrogen peroxide mist M in an amount of 10 µL to 50 µL per 500 mL bottle to deposit on the container 10.

In addition, when the amount of use of hydrogen peroxide is converted to the amount of gas G, it has been necessary to blow hydrogen peroxide gas G at a gas concentration of 5 mg/L to 10 mg/L toward the container 10 to sterilize the container 10 without sterilizing the preform 10a. However, when preliminary sterilization involving preheating of the preform 10a is performed as in this embodiment, commercial aseptic filling is enabled by blowing hydrogen peroxide gas G at a gas concentration of 1 mg/L to 5 mg/L, in both the case of the first sterilization step shown in Fig. 7(G1) or Fig. 7(G2) and the case of the second sterilization step shown in Fig. 8(I).

After the sterile water rinsing using warm water, or, when the sterile water rinsing is omitted, after the air rinsing, as shown in Fig. 9(L), the container 10 is aseptically filled, in an aseptic state, with liquid content L from a filling nozzle 110 of a filler 139 (a content filling step). Next, as shown in Fig. 9(M), the mouth portion 11 is sealed with a cap 103, which is a lid, by a capper 140, thereby obtaining a liquid-filled container 10A.

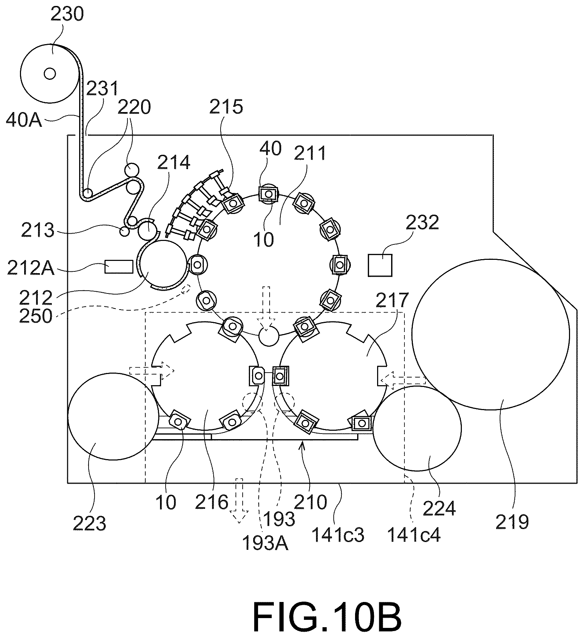

A content filling system 1 for aseptically filling the containers 10 sterilized by using hydrogen peroxide with content is configured as shown in Figs. 10A and 10B, for example.

As shown in Figs. 10A and 10B, the content filling system includes a preform feeder 111 that sequentially feeds, at predetermined intervals, bottomed tubular preforms 10a each having a mouth portion 11a (see Fig. 5(A1)), and the blow molding machine 112. The sterilant supply nozzles 193 (first sterilization part) that sterilize the container 10 molded by the blow molding machine 112, the labeling unit 210 that affixes the label 40 to the container 10, the sterilant supply nozzles 193 (second sterilization part) that sterilize the container 10 to which the label 40 has been affixed, and a content filling unit 113 that fills the container 10 with liquid content L and seals the container 10 with a cap 103 are provided in the system.

In the content filling system 1, portions from the sterilant supply nozzle 106, the blow molding machine 112 to the content filling unit 113 are enclosed by the chambers 141a, 141b, 141c1, 141c3, 141c2, 141g, 141d, 141e, and 141f.

The sterilant supply nozzles 193 (second sterilization part) constitute a sterilizer 188 together with the nozzle 145 that performs air rinsing and the nozzle 146 that performs sterile water rinsing.

The chamber 141a houses the sterilant supply nozzle 106 that supplies sterilant to the preform 10a, and the chamber 141b houses the blow molding machine 112 that molds the containers 10 and wheels 119, 121, and 122. The chamber 141c1 houses the sterilant supply nozzles 193A, the chamber 141c3 houses the labeling unit 210, and the chamber 141c2 houses the sterilant supply nozzles 193. The chamber 141g houses the wheels 191, 192, and 123, and the chamber 141d houses the content filling unit 113. The chamber 141e houses wheels 126 and 127, and the chamber 141f constitutes a discharge portion.

The content filling system 1 is preferably disposed in a building where the cleanliness of air is controlled, and more preferably disposed in a room maintained at slightly positive by a medium-efficiency filter. By supplying HEPA-filtered air to the chamber 141d, which houses the content filling unit 113, the positive pressure in the chamber 141d is made the highest, the positive pressure in the chambers 141e and 141f is next highest, and the chambers 141d to 141f are maintained at positive pressure. The chambers 141b and 141c3 are maintained as clean rooms. In order to create clean rooms, the chambers 141b and 141c3 have been supplied with sterile, HEPA-filtered positive-pressure air before the container 10 is manufactured. As a result, the interiors of the chambers 141b and 141c3 are maintained in a clean state (aseptic state), enabling the manufacture of the containers 10 with a high level of sterility. The sterile, HEPA-filtered positive-pressure air may also be supplied into the chambers 141a, 141c1, and 141c2. At the same time, the chambers 141a, 141c1, and 141c2 are forcibly evacuated as described below.

Before sterile, positive-pressure air is blown into all of the chambers 141b, 141c3, 141g, 141d, 141e, 141f, 141a, 141c1, and 141c2, the interiors of all the chambers 141b, 141c3, 141g, 141d, 141e, 141f, 141a, 141c1, and 141c2 may be sterilized with hydrogen peroxide gas at a concentration of 10 mg/L or less, or the outer surfaces of the chambers may be sterilized by SOP. In addition, for the chambers 141g, 141d, 141e, and 141f, in which droplets of the product liquid scatter and which have a high level of sterility, it is preferable to clean the outer surfaces of the chambers by COP before SOP. Portions with which the preforms 10a and the containers 10 come into contact may be irradiated with a UV lamp (ultraviolet sterilization). Alternatively, portions such as the mold 104, the stretch rod 105, and the gripper 132 with which the materials come into contact may be sterilized with ethanol or an agent containing 1% hydrogen peroxide. Of all the chambers 141b, 141c3, 141g, 141d, 141e, 141f, 141a, 141c1, and 141c2, the chamber 141a corresponding to the sterilant supply nozzle 106, the chamber 141b corresponding to the blow molding machine 112, and the chamber 141c3 corresponding to the labeling unit 210 do not necessarily need to be aseptic chambers to which sterile air is supplied.

Between the preform feeder 111 and the content filling unit 113, there are provided a preform conveying means that conveys the preforms 10a along a first conveying path, and a mold conveying means that conveys the mold 104 with a cavity 104c having the shape of the finished containers 10 along a second conveying path connected to the first conveying path. Furthermore, there is provided a container conveying means that performs sterilization, labeling, filling, etc., on the container 10 while conveying the container 10 molded by the mold 104 along a third conveying path connected to the second conveying path.

The first conveying path of the preform conveying means, the second conveying path of the mold conveying means, and the third conveying path of the container conveying means communicate with each other. The gripper 132 and the like that hold and convey the preforms 10a and the containers 10 are provided along these conveying paths.

The preform conveying means includes a preform conveyor 114 that sequentially feeds the preforms 10a, supplied from the preform feeder 111, to the first conveying path thereof at predetermined intervals. The preform conveying means also includes a row of wheels 204 and 205 that receive the preforms 10a at the end of the preform conveyor 114 and convey the preforms 10a, and an endless chain 118 that receives and conveys the preforms 10a.

In this case, the wheel 204 conveys the preforms 10a, and the wheel 204 is provided with the sterilant supply nozzle 106 that blows sterilant to the inner surface and outer surface of the preform 10a (see Fig. 5(A)).

The wheel 205 conveys the preforms 10a, and the wheel 205 is provided with the air nozzle 180 that blows hot air into the preforms 10a.

As shown in Fig. 10A, at fixed positions in the traveling path of the preforms 10a in the wheel 204, a sterilant gas generator that generates hydrogen peroxide gas G and the sterilant supply nozzle 106, as shown in Fig. 5(A), that discharges hydrogen peroxide gas G toward the preforms 10a are disposed.

Furthermore, in the traveling path of the preforms 10a in the wheel 205, the air nozzle 180 (see Fig. 5(B)) that discharges hot air P toward the preforms 10a to activate the hydrogen peroxide deposited on the inner and outer surfaces of the preforms 10a and to cause the hydrogen peroxide to be discharged to the outside of the preforms 10a is disposed. Here, in consideration of the sterility required for the product and the growth characteristics of microorganisms depending on the pH of the liquid content, the presence or absence of nitrogen sources, the presence or absence of carbonic acid, and the like, if there are no problems with the required level of sterilization for the preforms 10a and residual hydrogen peroxide affecting the container capacity, the preforms 10a may be conveyed to the heating furnace 133 without the hot air P being blown.

As shown in Fig. 10A, the wheel 204 is enclosed by the chamber 141a. The chamber 141a is connected to an exhaust means including a filter 136 for decomposing sterilant, such as hydrogen peroxide, in the air in the chamber 141a, and a blower 137. This prevents hydrogen peroxide from flowing into the adjacent blow molding machine 112. In the first conveying path, between a wheel 117, which is in contact with the wheel 205, to the wheel 119, which is in contact with the second conveying path, the heating furnace 133 that heats the preforms 10a to a molding temperature is provided.

The preforms 10a are uniformly heated while traveling through the heating furnace 133, and are heated to 90 °C to 130 °C, which is a temperature suitable for blow molding, except for the mouth portions 11a. The temperature of the mouth portions 11a is kept at 70 °C or less, at which deformation or the like does not occur, so as to maintain the airtightness when caps 103 are put thereon.

The blow molding machine 112 is disposed around the second conveying path. The blow molding machine 112 receives the preforms 10a heated in the heating furnace 133 and molds the containers 10.

A tunnel-shaped cover 186 (see Fig. 6(D)) that covers the preforms 10a running around the wheel 119 from above the mouth portions 11a is provided above the wheel 119, which is located between the first conveying path of the preform conveying means and the second conveying path of the mold conveying means. The sterile air Q is blown into the cover 186, toward the mouth portions 11a of the preforms 10a. The sterile air Q may be a part of the sterile air P supplied from the sterile air supply device.

With this structure, the preforms 10a enclosed by the chamber 141b, which serves as a clean room, are further covered by the cover 186 covering the sterile air Q, and are directed to the blow molding machine 112 while being maintained in a highly sterilized state.

The blow molding machine 112 has the molds 104 disposed along the wheel 120, and the molds 104 are opened at a position where the molds come into contact with the wheel 121, which is the starting end of the third conveying path. Containers are received by the grippers 132 around the wheel 121.

The containers 10 exiting the blow molding machine 112 and reaching the wheel 121 are inspected for the presence or absence of molding defects or the like by an inspection device 135 disposed on the outer periphery of the wheel 121 as necessary.

Of the inspected containers 10, rejected containers are removed from the conveying path by a rejecting device (not shown), and only accepted containers are conveyed to the wheel 122.

In the third conveying path, a tunnel-shaped cover 187 (see Fig. 6(F)) that covers the containers 10 from above the mouth portions 11a is provided above the traveling paths of the containers 10 in the wheels 121 and 122. The sterile air Q blown into the cover 187 may be a part of the sterile air P supplied from the sterile air supply device.

A wheel 189 that conveys the containers 10 is connected to the downstream side of the wheel 122, and the above-described sterilant supply nozzles 193A are provided at the wheel 189. The wheel 189 and the sterilant supply nozzles 193A constitute the first sterilization part and are housed in the chamber 141c1 (first sterilization part aseptic chamber).

The labeling unit 210 is disposed downstream of the wheel 189 and the sterilant supply nozzles 193A. The labeling unit 210 includes the star wheel 211, the label transport drum 212, the adhesive application device 212A, and the label pressing device 215 including, for example, a brush. The labeling unit 210 is disposed in the chamber 141c3 (labeling unit aseptic chamber) disposed between the chamber 141c1 and the chamber 141c2.

Next, the labeling unit 210 will be described in detail with reference to Figs. 10A and 10B. As shown in Figs. 10A and 10B, the labeling unit 210 includes a label feeder 230 that feeds a band-shaped label 40A, a rotary cutter 213 that cuts the band-shaped label 40A fed from the label feeder 230 to produce labels 40 to be affixed to the containers 10, an upstream label transport drum 214 that conveys the labels 40 produced by the rotary cutter 213 while holding by suction the labels 40, a label transport drum 212 that holds by suction the labels 40 conveyed from the upstream label transport drum 214 and conveys the labels 40, and the adhesive application device 212A that applies an adhesive, such as hot melt, to the labels 40 held by suction by the label transport drum 212. Among these, the upstream label transport drum 214 and the label transport drum 212 include a suction mechanism that sucks the labels 40.

The labels 40 conveyed by the label transport drum 212 are affixed to the outer peripheries of the body portions 20 of the containers 10 conveyed by the star wheel 211.

Holding portions (not shown) that rotatably hold the containers 10 are provided on the outer periphery of the star wheel 211. The containers 10 conveyed from the wheel 189 are held by the holding portions of the star wheel 211 via a conveying wheel 223 and an inlet wheel 216. In the inlet wheel 216, the pitch between the containers 10 may be changed as appropriate so that the labels 40 do not overlap (interfere) with each other at the label transport drum 212. When the containers 10 are polygonal containers, such as square bottles, and require adjustment of the label attachment position, a device for regulating the orientation of the containers 10 may be provided between the inlet wheel 216 and the star wheel 211. The labels 40 held by suction by the label transport drum 212 are transferred to the containers 10 held by the holding portions on the outer periphery of the star wheel 211. Thereafter, as the label transport drum 212 rotates and the holding portions of the star wheel 211 rotate, the labels 40 are wound around and affixed to the outer peripheries of the containers 10 via the applied adhesive. Instead of affixing the labels 40 to the outer peripheries of the containers 10 via an adhesive, thermal labels may be used, and the thermal labels may be affixed to the outer peripheries of the containers 10 while heating the labels.

When empty containers 10 are conveyed at a high speed, the containers 10 are subjected to the influence of centrifugal force. In order to accurately wind the labels 40, it is necessary to fix the upper end faces of the mouth portions 11 and/or the support portions 17 of the containers 10 so that the containers 10 remain level. In this case, conical or columnar jigs may be inserted into the mouth portions 11, or the gate portions 35 at the center of the bottom portions 30 of the containers 10 may be pressed from below so that the containers 10 remain level. When empty containers 10 are thin-walled bottles, the labels 40 may be wound in a state in which air (preferably, sterile air filtered by a sterilizing filter) is supplied from the upper end faces of the mouth portions 11 of the containers 10, so that the internal pressure of the containers 10 is slightly positive. Hydrogen peroxide gas (5 to 300 mg/L) may be added to the sterile air to sterilize the inner surfaces of the containers 10 at the same time. In addition, when air is supplied from the mouth portions 11 of the containers 10, a leak inspection of the containers 10 may be performed at the same time. In particular, the upper end faces of the mouth portions 11 of the containers 10 are sealed with air supply nozzles, a pressure is applied to the inside of the containers 10 for a certain period of time, and a decrease in pressure (reduction rate) is measured to check for pinholes in the containers 10.

Thereafter, the containers 10 held by the holding portions of the star wheel 211 are conveyed to the label pressing device 215. The labels 40 wound around the containers 10 are pressed against the containers 10 by the label pressing device 215.

The labels 40 wound around the containers 10 are pressed by the label pressing device 215 and are accurately and reliably affixed to the containers 10. In particular, the ends of the labels 40 are reliably brought into close contact with each other by the label pressing device 215.

The containers 10 to which the labels 40 have been affixed are conveyed via an outlet wheel 217, a connecting wheel 224, and a discharge wheel 219 to a next wheel 190. In the outlet wheel 217, as in the inlet wheel 216, the pitch between the containers 10 may be changed in accordance with the subsequent sterilization, filling and capping steps.

The labels 40 that have been affixed to the containers 10 are inspected for the attachment state (e.g., attachment position) by the inspection device 232 while the containers 10 are at the star wheel 211 and/or the outlet wheel 217, and containers 10 with labels 40 affixed in a defective state are discharged from the discharge mechanism 255. The inspection device 232 may simultaneously inspect the mouth portions 11, the support portions 17, and the bottom portions 30 of the containers 10.

Of the labeling unit 210 shown in Figs. 10A and 10B, the conveying wheel 223, the inlet wheel 216, the star wheel 211, the outlet wheel 217, the connecting wheel 224, the inspection device 232, the rotary cutter 213, the upstream label transport drum 214, the label transport drum 212, the adhesive application device 212A, and conveying rollers 220 are all housed in the chamber 141c3 (labeling unit aseptic chamber). The label feeder 230 is disposed outside the chamber 141c3. The band-shaped label 40A fed from the label feeder 230 is fed into the chamber 141c3 through an opening 231, passes through the conveying rollers 220, and is cut by the rotary cutter 213 to produce the labels 40.

In the third conveying path, the labeling unit 210 is provided downstream of the wheel 189. The wheels 190, 191, 192, and 123 are connected to the labeling unit 210, and the sterilant supply nozzles 193 (second sterilization part) (see Fig. 8(I)) and the sterile air supply nozzle 145 (see Fig. 8(J1) or (J2)) are provided in the row of the wheels 190, 191, 192, and 123.

In particular, a plurality of (three in Fig. 10A) sterilant supply nozzles 193 are disposed at fixed positions in the traveling path of the containers 10 around the wheel 190. Furthermore, a tunnel 144 (see Fig. 8(I)) through which the containers 10 pass is also disposed corresponding to the sterilant supply nozzles 193. The hydrogen peroxide mist M, gas G, or a mixture thereof discharged from the sterilant supply nozzles 193 enters the containers 10 and deposit, in the form of a thin film, on the inner surfaces of the containers 10. The hydrogen peroxide mist M, gas G, or a mixture thereof flows along the outer surfaces of the containers 10 and the outer surfaces of the labels 40, fills the tunnel 44, and deposits, in the form of a thin film, on the outer surfaces of the containers 10 and the outer surfaces of the labels 40.

One or more sterile air supply nozzles 145 and nozzles 146 are disposed at fixed positions in the traveling path of the containers 10 around the wheel 192. The sterile air N discharged from the sterile air supply nozzle 145 comes into contact with the inner surfaces and outer surfaces of the containers 10 and the outer surfaces of the labels 40 to remove the film of the excess hydrogen peroxide solution deposited on the surfaces of the containers 10. When the sterile air N is hot air, the hydrogen peroxide deposited on the inner surfaces and outer surfaces of the containers 10, the outer surfaces of the labels 40, and the spaces between the outer surfaces of the containers 10 and the inner surfaces of the labels 40 is activated to enhance the sterilization effect. Thus, any microorganisms adhering to the gaps between the labels 40 and the containers 10 can be reliably killed, and the sterility of the aseptic chambers 141g, 141d, 141e, and 141f can be maintained for a long time.

The sterilant supply nozzles 193 and the sterile air supply nozzles 145 may be arranged in large numbers around the wheels 190 and 192, at the same pitch as the pitch between the containers 10, and may be configured to blow the hydrogen peroxide gas G and the sterile air N into the containers 10 while being turned in synchronization with the wheels 190 and 192.

In the third conveying path, the filler 139 (content filling unit) and the capper 140 are provided at positions between the wheel 124, which is in contact with the wheel 123, and the wheel 127.

In particular, the filler 139 is formed by providing a large number of filling nozzles 110 (see Fig. 9(L)) for filling the containers 10 with liquid content L around the wheel 124. The capper 140 that attaches caps 103 (see Fig. 9(M)) to the containers 10 filled with liquid content L to seal the containers 10 is provided around the wheel 126.

In the first to third conveying paths, the wheel 204 is enclosed by the chamber 141a. The area starting from the wheel 205, the wheel 117 to the wheel 122 and the vicinity thereof are enclosed by the chamber 141b. The wheel 189 and the vicinity thereof are enclosed by the chamber 141c1. The conveying wheel 223, the inlet wheel 216, the star wheel 211, the outlet wheel 217, the connecting wheel 224, the discharge wheel 219, the rotary cutter 213, the upstream label transport drum 214, the label transport drum 212, and the adhesive application device 212A of the labeling unit 210 are enclosed by the chamber 141c3. The wheel 190 and the vicinity thereof are enclosed by the chamber 141c2. The area starting from the wheel 191 to the wheel 123 and the vicinity thereof are enclosed by the chamber 141g. The wheel 124, the wheel 125, and the vicinity thereof are enclosed by the chamber 141d, and the area starting from the wheel 125 to the wheel 127 and the vicinity thereof are enclosed by the chamber 141e.

Of these chambers, the chamber 141c1 encloses the sterilant supply nozzles 193A (first sterilization part) and constitutes the first sterilization part chamber. The chamber 141c2 encloses the sterilant supply nozzles 193 (second sterilization part) and constitutes the second sterilization part chamber.

The chamber 141c3 encloses the labeling unit 210 including the conveying wheel 223, the inlet wheel 216, the star wheel 211, the outlet wheel 217, the connecting wheel 224, the discharge wheel 219, the rotary cutter 213, the upstream label transport drum 214, the label transport drum 212, and the adhesive application device 212A and constitutes a labeling unit chamber.

As described above, sterile, positive-pressure air is supplied into the chambers 141b, 141c3, 141g, 141d, 141e, and 141f to maintain the interiors of the chambers 141b, 141c3, 141g, 141d, 141e, and 141f clean. The chambers 141a, 141c1, and 141c2 are evacuated, and the chambers 141a, 141c1, and 141c2 are maintained at -20 Pa to 5 Pa, preferably, -10 Pa to 1 Pa. Thus, the chambers 141a, 141c1, and 141c2 are maintained at a lower pressure than the chambers 141b and 141c3 with an internal pressure of 0 to 10 Pa, and than the chambers 141g, 141d, 141e, and 141f with an internal pressure of 10 to 100 Pa. In particular, the chamber 141c3 is constantly supplied with sterile air filtered by a HEPA filter or the like (not shown) so as to have a positive pressure relative to the adjacent chambers 141c1 and 141c2.

As described, the chamber 141c3 (labeling unit aseptic chamber) enclosing the labeling unit 210 has a positive pressure relative to the chamber 141c1 (first sterilization part aseptic chamber) enclosing the sterilant supply nozzles 193A and the chamber 141c2 (second sterilization part aseptic chamber) enclosing the sterilant supply nozzles 193. Hence, the hydrogen peroxide in the chamber 141c1 or the hydrogen peroxide in the chamber 141c2 does not enter the chamber 141c3 enclosing the labeling unit 210. This prevents the hydrogen peroxide from interfering with the operation of the labeling unit 210.

In this embodiment, it is preferable that the chamber 141b be at a positive pressure relative to the chamber 141c1. By setting so, it is possible to prevent the hydrogen peroxide in the chamber 141c1 from entering the chamber 141b, and the hydrogen peroxide from adversely affecting a blow-molding step.

Sterile air purified by a HEPA filter or the like (not shown) is constantly supplied into the chamber 141b. This makes the chamber 141b a clean room, preventing entry of microorganisms.

The interiors of all the chambers 141b, 141c3, 141g, 141d, 141e, 141f, 141a, 141c1, and 141c2 are sterilized by, for example, cleaning outside of place (COP) and sterilizing outside of place (SOP), and then, sterilant or cleaner gas or mist is discharged from all the chambers 141b, 141c3, 141g, 141d, 141e, 141f, 141a, 141c1, and 141c2 by an exhaust means disposed in each of the chambers 141b, 141c3, 141g, 141d, 141e, 141f, 141a, 141c1, and 141c2 or an exhaust unit disposed commonly to these chambers. As a result of sterile air purified by a scrubber, a filter, or the like (not shown) being supplied into the chambers 141b, 141c3, 141g, 141d, 141e, 141f, 141a, 141c1, and 141c2, the sterility of the chambers 141b, 141c3, 141g, 141d, 141e, 141f, 141a, 141c1, and 141c2 is maintained. Of all the chambers above, COP and SOP are always performed for the chambers 141g, 141d, 141e, and 141f, whereas COP and SOP need not necessarily be performed for the chambers 141a, 141b, 141c1, 141c2, and 141c3. Furthermore, of all the chambers 141b, 141c3, 141g, 141d, 141e, 141f, 141a, 141c1, and 141c2, the chambers 141a, 141b, 141c1, 141c2, and 141c3 need not necessarily be made as aseptic chambers to which sterile air is supplied.

The chamber 141c1 functions as an atmosphere isolation chamber that isolates the atmosphere in the chamber 141b from the atmosphere in the chamber 141c3. An exhaust means similar to the above-described exhaust means is also connected to the chamber 141c1, and the air inside the chamber 141c1 is forcibly discharged to the outside. The chamber 141c1 is at a negative pressure relative to the chamber 141b. This can prevent the atmosphere in the chamber 141c3, including the cleaning agent gas generated by COP and SOP in the chamber 141d and the sterilant mist generated in the chamber 141c2, from flowing into the chamber 141b of the blow molding machine 112 through the chamber 141c1.

The required levels of sterilization for the environments (the chambers and the product liquid lines) in the container sterilizing apparatus and for the content of the liquid-filled container 10A vary with the pH of the content and the amount of nitrogen sources, carbon sources, catechin, and the like contained therein.

Accordingly, the details of COP and SOP and the areas to be subjected to COP and SOP vary depending on the content. In addition, a sterile air supply pipe and bottle sterilization may be unnecessary depending on the product.

Next, an operation of the content filling system, that is, the content filling method will be described with reference to Figs. 5 to 11.

First, as shown in Fig. 5(A), preforms 10a are fed from the preform feeder 111, and the preforms 10a are delivered to the wheel 204 via the preform conveyor 114.

The preforms 10a are conveyed by the wheel 204, during which hydrogen peroxide gas G, mist, or a mixture thereof is supplied to the inner surfaces and the outer surfaces of the preforms 10a from the sterilant supply nozzle 106.

Next, the preforms 10a, to the inner surfaces and the outer surfaces of which the hydrogen peroxide gas G, mist, or a mixture thereof has been supplied, are conveyed to the wheel 205. While the preforms 10a are rotationally conveyed by the wheel 205, hot air P is blown from the air nozzle 180 into the preforms 10a (see Fig. 5(B)). The heat of the hot air P activates the hydrogen peroxide deposited on the inner surfaces of the preforms 10a, killing the microorganisms on the preforms 10a. Furthermore, the hot air P removes the excess hydrogen peroxide from the preforms 10a.

Thereafter, the spindles 143 (see Fig. 5(C)) on the endless chain 118 receive the preforms 10a and convey the preforms 10a into the heating furnace 133.

In the heating furnace 133, the preforms 10a are heated by the infrared heaters 118a, and the entire preforms 10a except for the mouth portions 11a are uniformly heated to a temperature range suitable for blow molding. During this, the entire preforms 10a are heated, and the hydrogen peroxide remaining on the outer surfaces of the preforms 10a is activated, whereby the outer surfaces of the preforms 10a are sterilized.

The preforms 10a, heated to a molding temperature in the heating furnace 133, are subjected to blowing of sterile air Q while traveling around the wheel 119 through the interior of the cover 186 (see Fig. 6(D)). This allows the preforms 10a to be conveyed to the blow molding machine 112 while maintaining sterility. When the sterile air Q is hot air, the preforms 10a reach the blow molding machine 112 while maintaining a temperature suitable for molding.

When passing the outer periphery of the wheel 120, the preforms 10a are inserted into the molds 104, as shown in Fig. 6(E), and are expanded into finished containers 10 in the cavities 104c by sterile high-pressure air blown therein.

After the molds 104 are opened, the molded containers 10 are taken out of the molds 104 by the grippers around the wheel 121, and are inspected by the inspection device 135 for the presence or absence of molding defects or the like.

Defective containers 10 are removed from the line by the discharge device (not shown). Only good containers 10 are delivered to the wheel 122 and then conveyed to the wheel 189.

Furthermore, the containers 10 are subjected to blowing of sterile air Q while traveling from the wheel 121 to the wheel 122 through the interior of the cover 187 (see Fig. 6(F)). This allows the containers 10 to be conveyed to the wheel 189 while maintaining sterility. When the sterile air Q is hot air, the containers 10 reach the wheel 189 while appropriately maintaining a temperature suitable for sterilization.

When the containers 10 reach the wheel 189, the containers 10 travel around the wheel 189. During this, the hydrogen peroxide mist M, gas G, or a mixture thereof from the sterilant supply nozzles 193A (first sterilization part) is blown onto at least the outer surfaces, and additionally the inner surfaces, of the containers 10 for sterilization (see Figs. 7(G1) and 7(G2)).

The step of blowing the hydrogen peroxide mist M, gas G, or a mixture thereof from the sterilant supply nozzles 193A in this way is the first sterilization step. In the first sterilization step, the hydrogen peroxide solution mist M, gas G, or a mixture thereof may be blown only onto the outer surfaces of the containers 10, rather than to both the inner surfaces and outer surfaces of the containers 10.

The hydrogen peroxide aqueous solution, vaporized at a temperature at or above its boiling point and in a gaseous state or in a mist form, is supplied toward the containers 10. The amount of the hydrogen peroxide gas or mist that condenses and deposits on at least the outer surface of each container 10, in terms of 35% by mass, is preferably in the range of 0.01 µL/cm² or more and 0.1 µL/cm² or less. By setting the amount of deposited hydrogen peroxide to 0.01 µL/cm² or more, uniform sterilization effect can be achieved across the container 10. If the amount of deposited hydrogen peroxide is 0.1 µL/cm² or more, the hydrogen peroxide consumption and the required exhaust airflow increase, which is not cost-effective. More preferably, the amount of the hydrogen peroxide condensate film deposited on at least the outer surfaces of the containers 10, in terms of 35 % by mass, is 0.03 µL/cm² or more and 0.07 µL/cm² or less. Preferably, the area of the outer surface of the container 10 on which a hydrogen peroxide condensate film is to be deposited is at least the entire area to which the label is to be affixed. Alternatively, the area may be limited to a region extending, in the vertical direction in which the label is affixed, 0 mm to 30 mm away from a position where the label is not affixed.

The container 10, to which the hydrogen peroxide has been supplied from the sterilant supply nozzles 193A, is then conveyed to the labeling unit 210. The hydrogen peroxide deposited on the outer surface of the container 10 may be removed by sterile air at the position where the container 10 is conveyed to the labeling unit 210. In this case, the hydrogen peroxide may be activated by hot air.

The labeling step performed by the labeling unit 210 shown in Figs. 10A and 10B will be described below.

As shown in Figs. 10A and 10B, in the labeling unit 210, the band-shaped label 40A is fed from the label feeder 230. The band-shaped label 40A from the label feeder 230 is cut by the rotary cutter 213 to produce the labels 40.

Next, the labels 40 produced by the rotary cutter 213 are transferred via the upstream label transport drum 214 to the label transport drum 212, and are held by suction and conveyed by the label transport drum 212. While the labels 40 are held by suction and conveyed by the label transport drum 212, the adhesive application device 212A applies an adhesive (or glue), such as hot melt, to the labels 40.

Thereafter, the labels 40, which are held by suction on the label transport drum 212 and provided with an adhesive by the adhesive application device 212A, are affixed to the outer peripheries of the body portions 20 of the containers 10 conveyed by the star wheel 211.

The holding portions (not shown) that rotatably hold the containers 10 are provided on the outer periphery of the star wheel 211, and the containers 10 delivered by the wheel 189 are held by the holding portions of the star wheel 211 via the conveying wheel 223 and the inlet wheel 216. The labels 40 held by suction on the label transport drum 212 and provided with an adhesive by the adhesive application device 212A are then transferred to the containers 10 held by the holding portions on the outer periphery of the star wheel 211. Thereafter, as the label transport drum 212 rotates and the holding portions of the star wheel 211 rotate, and the labels 40 are wound around and affixed to the outer peripheries of the containers 10.

Thereafter, the containers 10 held by the holding portions of the star wheel 211 are conveyed to the label pressing device 215. Next, the labels 40 wound around the containers 10 are pressed against the containers 10 by the label pressing device 215.

The labels 40 wound around the containers 10 are pressed by the label pressing device 215 and are accurately and reliably affixed to the containers 10. In particular, the ends of the labels 40 are reliably brought into close contact with each other by the label pressing device 215.

The containers 10 to which the labels 40 have been affixed are conveyed via the outlet wheel 217, the connecting wheel 224, and the discharge wheel 219 to the next wheel 190.

During this, the labels 40 that have been affixed to the containers 10 are inspected for the attachment state (e.g., attachment position) by the inspection device 232, and containers 10 with labels 40 affixed in a defective state are discharged from the discharge mechanism 255 via the wheel 224. Note that the filler 139 fills the containers 10 with liquid content L to produce liquid-filled containers 10A, without the inspection device 232 inspecting the attachment state of the labels 40. The attachment state of the labels 40 may be inspected simultaneously when a liquid level inspection is performed on the liquid-filled containers 10A or when an inspection of the caps 103 is performed.

Next, the containers 10 are conveyed to the wheel 190, and the containers 10, while traveling around the wheel 190, are sterilized by the hydrogen peroxide mist M, gas G, or a mixture thereof blown from the sterilant supply nozzles 193, as shown in Fig. 8(I). In this case, the inner surfaces and outer surfaces of the containers 10 and the outer surfaces of the labels 40 are sterilized by the hydrogen peroxide mist, gas G, or a mixture thereof blown from the sterilant supply nozzles 193 (second sterilization part) (second sterilization step). Subsequently, sterile air N is blown onto the containers 10 traveling around the wheel 192 as shown in Fig. 8(J1) or (J2), whereby the containers 10 are air-rinsed.

In another embodiment, as shown in Fig. 10B, the sterilant supply nozzles 193A may be provided at the wheel 216, the sterilant supply nozzles 193 may be provided at the wheel 217, and the wheel 216 and the wheel 217 may be disposed in a sterilant spraying chamber 141c4 (see the broken line in Fig. 10B). In this case, the chamber 141c3, the chamber 141c1 that houses the wheel 189, and the chamber 141c2 that houses the wheel 190 can be formed as a single component. Furthermore, the wheel 189 at which the sterilant supply nozzles 193A are provided, the wheel 223, and the wheel 190 at which the sterilant supply nozzles 193 are provided, and the wheel 191 can be eliminated. By eliminating the wheels 189, 223, 190, and 191, the number of wheels can be reduced, making the overall system more compact. In this case, to prevent the sterilant in the sterilant spraying chamber 141c4 from flowing out toward the star wheel 211, it is preferable that the sterilant spraying chamber 141c4 be maintained at a negative pressure relative to the chamber 141c3, so that the pressure difference between the sterilant spraying chamber 141c4 and the chamber 141c3 is 10 Pa or more. In addition, after sterilant (particularly, hydrogen peroxide gas, mist, or a mixture thereof) is sprayed, if the sterilant condensing and depositing on the outer surface of the container affects the label attachment, the sterilant may be vaporized by sterile hot air. The sterilant may be vaporized in a part of the area where an adhesive such as hot melt is applied to the container.

Thereafter, the containers 10 pass through the wheel 123 and reach the content filling unit 113 in the chamber 141d.

As shown in Fig. 9(L), in the content filling unit 113, the containers 10 are filled with liquid content (also referred to as content) L that has been sterilized in advance through the filling nozzle 110 of the filler 139 (content filling unit). The containers 10 filled with the liquid content L are sealed with the caps 103 applied by the capper (also referred to as a sealing unit) 140 disposed in the chamber 141e (see Fig. 9(M)), and are discharged to the outside through the outlet of the chamber 141f.

In this embodiment, the step of allowing the preforms 10a to adsorb the sterilant is performed in-line with the blow molding machine, but may be performed off-line as long as the state in which a part of the sterilant component is adsorbed can be maintained during blow molding.

As described above, according to this embodiment, the labeling unit 210 is provided between the sterilant supply nozzles 193A (first sterilization part) that blow hydrogen peroxide onto the containers 10 and the sterilant supply nozzles 193 (second sterilization part) that blow hydrogen peroxide onto the containers 10 to which the labels 40 have been affixed, and the labeling unit 210 affixes the labels 40 to the body portions 20 of the containers 10. By incorporating the labeling unit 210 upstream of the filler 139 in this way, the overall system can be made compact compared with a case where the labeling unit 210 is provided downstream of the filler 139, for example.

That is, if the labeling unit 210 is provided downstream of the filler 139, the containers 10 have already been filled with liquid content. Hence, labels are affixed to the containers 10 while the containers 10 filled with liquid content are being conveyed. The operation of conveying the containers 10 filled with liquid content is not easy, and the accuracy of label attachment to the containers 10 filled with liquid content decreases. Hence, a large buffer area for containers is sometimes provided upstream of the labeling unit, in anticipation of a failure of the labeling unit. This increases the installation area of the overall system. In addition, the outer surfaces of the filled containers 10 are often wet with cleaning water. In order to avoid label attachment defects, water droplets on the outer surfaces of the containers 10 need to be removed with air or the like prior to labeling. In addition, because the pitch between the containers 10 discharged from the filler 139 is irregular, the containers 10 need to be aligned into a single row at the inlet of the labeling unit, and the pitch needs to be adjusted such that the containers 10 are at equal intervals, using a screw or the like. When the size and shape of the containers 10 vary, the pitch-adjusting screw needs to be replaced each time.

In contrast, according to this embodiment, the labeling unit 210 is provided between the sterilant supply nozzles 193A (first sterilization part) and the sterilant supply nozzles 193 (second sterilization part). Hence, the labels 40 can be affixed, by the labeling unit 210, with high accuracy to the containers 10 that are not yet filled with liquid content. Furthermore, because no buffer area is required upstream of the labeling unit 210, the overall content filling system can be made compact.

In addition, hydrogen peroxide is blown onto the inner surfaces and outer surfaces of the containers 10 by the sterilant supply nozzles 193A (first sterilization part), the labels 40 are affixed to the outer surfaces of the containers 10 by the labeling unit 210, and then hydrogen peroxide is blown onto the inner surfaces and outer surfaces of the containers 10 and the outer surfaces of the labels 40 by the sterilant supply nozzles 193 (second sterilization part).

In this way, hydrogen peroxide can be blown onto the inner surfaces and outer surfaces of the containers 10 and the outer surfaces of the labels 40, whereby microorganisms on the inner surfaces and outer surfaces of the containers 10 and the outer surfaces of the labels 40 can be killed.

Thereafter, an air rinsing step is performed in which sterile air N is blown from the nozzle 145 onto the containers 10.

In this case, by blowing heated sterile air N from the nozzle 145, the hydrogen peroxide supplied by the sterilant supply nozzles 193 (second sterilization part) and deposited on the inner surfaces and outer surfaces of the containers 10 and the outer surfaces of the labels 40 can be activated. By doing so, microorganisms on the inner surfaces and outer surfaces of the containers 10 and the outer surfaces of the labels 40 can be reliably killed by the activated hydrogen peroxide.

At the same time, the hydrogen peroxide blown onto the containers 10 by the sterilant supply nozzles 193A (first sterilization part) and remaining between the outer surfaces of the containers 10 and the inner surfaces of the labels 40 can be activated. By doing so, microorganisms between the outer surfaces of the containers 10 and the inner surfaces of the labels 40 can be reliably killed by the activated hydrogen peroxide. In this case, at least the outer surfaces of the containers 10 or at least the inner surfaces of the labels 40 can be sterilized by the activated hydrogen peroxide.

In addition, the sterilant supply nozzles 193A are enclosed by the chamber 141c1 (first sterilization part aseptic chamber), the labeling unit 210 is enclosed by the chamber 141c3 (labeling unit aseptic chamber), and the sterilant supply nozzles 193 are enclosed by the chamber 141c2 (second sterilization part aseptic chamber).

In this case, the chamber 141c3 is at a positive pressure relative to the chamber 141c1 and the chamber 141c2. Hence, during the operation of the content filling system 1, the hydrogen peroxide in the chamber 141c1 and the chamber 141c2 does not enter the chamber 141c3, and thus, the hydrogen peroxide does not adversely affect the operation of the labeling unit 210 in the chamber 141c3.

The chamber 141b in which the blow molding machine 112 is disposed is at a positive pressure relative to the chamber 141c1. Hence, during the operation of the content filling system 1, the hydrogen peroxide in the chamber 141c1 does not enter the chamber 141b, and the hydrogen peroxide does not adversely affect the operation of the blow molding machine 112 in the chamber 141d. In this embodiment, an example has been described in which the sterilant is supplied to the outer surfaces of the containers 10, and then the labels 40 are affixed to the outer surfaces of the containers 10. However, the present invention is not limited thereto, and the labels 40 may be affixed to the outer surfaces of the containers 10 after the sterilant is applied to the labels 40. Alternatively, the labels 40 may be affixed to the outer surfaces of the containers 10 after the outer surfaces of the containers 10 are sterilized with sterilant and the labels 40 are sterilized with sterilant. Alternatively, the labels 40 may be affixed to the containers 10 after the outer surfaces of the containers 10 and the labels 40 are sterilized by applying ultraviolet light, radiation, heat, or the like to the outer surfaces of the containers 10 and the labels 40.

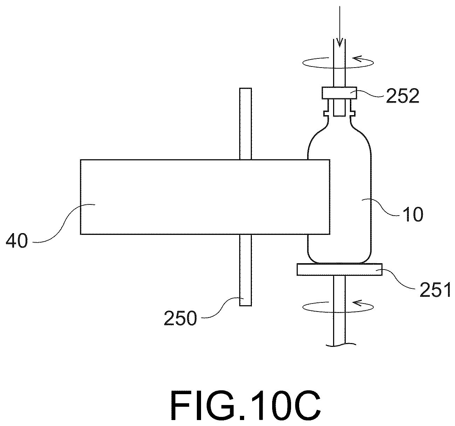

When ultraviolet sterilization is performed on the outer surfaces of the containers 10 and the labels 40 by using a UV lamp 250, it is preferable that the UV lamp 250 be disposed vertically so that the labels 40 to be affixed and the entire surfaces of the containers 10 (at least the surfaces to which the labels are to be affixed) are irradiated (see Fig. 10C). It is preferable that the UV lamps, which irradiate the body portions of the containers 10 with light while the containers 10 are rotated by the star wheel 211, be disposed in a number sufficient to irradiate the entire circumference of the containers 10. The UV lamp for sterilizing the surfaces of the labels to be affixed and the UV lamp for sterilizing the body portions of the containers 10 may be the same, or may be separately disposed. Alternatively, sterile air may be jetted from a fixed nozzle 252 into the containers 10 on a table 251. Ultraviolet sterilization is simultaneously performed on the labels 40 and the outer surfaces of the containers 10 using the UV lamp 250. Ultraviolet light is a type of electromagnetic radiation having wavelengths of 100 nm to 380 nm, and the band referred to as UV-C, with wavelengths of 100 nm to 280 nm, is particularly effective for sterilization. In addition, a wavelength of 253.7 nm exhibits the highest sterilization effect, and it is optimal for the ultraviolet light to include this wavelength. Furthermore, a low-pressure mercury lamp, a high-pressure mercury lamp, a xenon flash lamp, an ultraviolet LED, or the like may be used. Instead of ultraviolet light, electron beams may be used. In this embodiment, an example has been described in which the containers 10 to which the labels 40 have been affixed are sterilized by blowing sterilant thereon, but the present invention is not limited thereto. The containers 10 to which the labels 40 have been affixed may be sterilized by electron beam irradiation.

### Reference Signs List

1 content filling system
10 container
10A liquid-filled container
10a preform
40 label
40A band-shaped label
104 mold
106 sterilant supply nozzle
110 filling nozzle
111 preform feeder
112 blow molding machine
113 content filling unit
114 preform conveyor
118 endless chain
118a infrared heater
119, 120, 121, 122, 123, 124, 125, 126, 127 wheel139 filler140 capper
141a, 141b, 141c1, 141c2, 141c3, 141d, 141e, 141f, 141g chamber
143 spindle
144 tunnel
144A tunnel
145 nozzle
146 nozzle
180 air nozzle
186 cover
187 cover
193, 193A sterilant supply nozzle
204, 205 wheel
210 labeling unit
211 star wheel
212 label transport drum
212A adhesive application device
213 rotary cutter
214 upstream label transport drum
215 label pressing device
230 label feeder
250 UV lamp

## Claims

1. A content filling system comprising:
a first sterilization part configured to sterilize at least an outer surface of a container or at least an inner surface of a label;
a labeling unit configured to affix the label to the outer surface of the container sterilized by the first sterilization part;
a second sterilization part configured to sterilize the container to which the label has been affixed in the labeling unit;
a content filling unit configured to fill the container sterilized by the second sterilization part with content; and
a sealing unit configured to seal the container.

2. The content filling system according to claim 1, wherein an air rinse unit configured to blow hot air onto the container is provided between the second sterilization part and the content filling unit.

3. The content filling system according to claim 1, wherein the second sterilization part blows sterilant onto an inner surface and the outer surface of the container.

4. The content filling system according to claim 1, wherein the first sterilization part, the labeling unit, and the second sterilization part are enclosed by a first sterilization part chamber, a labeling unit chamber, and a second sterilization part chamber, respectively, so as to be partitioned from each other, and the labeling unit chamber for the labeling unit is at a positive pressure relative to the first sterilization part chamber for the first sterilization part and/or the second sterilization part chamber for the second sterilization part.

5. The content filling system according to claim 4, wherein the labeling unit includes: a label feeder configured to feed a band-shaped label; a rotary cutter configured to cut the band-shaped label fed from the label feeder to produce the label; a label transport drum that holds by suction the label produced by the rotary cutter; and a star wheel that conveys the container, receives the label held by the label transport drum, and affixes the label to the container, and wherein the star wheel, the label transport drum, and the rotary cutter are housed in the labeling unit chamber.

6. The content filling system according to claim 5, wherein an adhesive application device configured to apply an adhesive or glue to the label held on the label transport drum is provided.

7. The content filling system according to claim 5, wherein the label feeder is disposed outside the labeling unit chamber, and the band-shaped label is fed from the label feeder into the labeling unit chamber through an opening in the labeling unit chamber.

8. A content filling method comprising:
a first sterilization step of sterilizing, with a first sterilization part, at least an outer surface of a container;
a labeling step of affixing, in a labeling unit, a label to the outer surface of the container sterilized by the first sterilization part;
a second sterilization step of sterilizing, with a second sterilization part, the container to which the label has been affixed in the labeling unit;
a content filling step of filling, in a content filling unit, the container sterilized by the second sterilization part with content; and
a sealing step of sealing the container in a sealing unit.

9. The content filling method according to claim 8, wherein hot air is blown onto the container by an air rinse unit provided between the second sterilization part and the content filling unit.

10. The content filling method according to claim 8, wherein the second sterilization part blows sterilant onto an inner surface and the outer surface of the container.

11. The content filling method according to claim 8, wherein the first sterilization part, the labeling unit, and the second sterilization part are enclosed by a first sterilization part chamber, a labeling unit chamber, and a second sterilization part chamber, respectively, so as to be partitioned from each other, and the labeling unit chamber for the labeling unit is at a positive pressure relative to the first sterilization part chamber for the first sterilization part and/or the second sterilization part chamber for the second sterilization part.

12. The content filling method according to claim 11, wherein the labeling step includes: a step of feeding a band-shaped label from a label feeder; a step of cutting, with a rotary cutter, the band-shaped label fed from the label feeder to produce the label; a step of holding by suction and transporting, with a label transport drum, the label produced by the rotary cutter; and a step of conveying the container, receiving the label held by the label transport drum, and affixing the label to the container with a star wheel, and wherein the star wheel, the label transport drum, and the rotary cutter are housed in the labeling unit chamber.

13. The content filling method according to claim 12, wherein an adhesive is applied to the label held on the label transport drum by an adhesive application device.

14. The content filling method according to claim 12, wherein the label feeder is disposed outside the labeling unit chamber, and the band-shaped label is fed from the label feeder into the labeling unit chamber through an opening in the labeling unit chamber.
